Europäisches Patentamt

European Patent Office

Office européen des brevets

(11) Veröffentlichungsnummer: **0 249 877 B1**

# EUROPÄISCHE PATENTSCHRIFT

(45) Veröffentlichungstag der Patentschrift: **16.09.92**

(21) Anmeldenummer: **87108408.3**

(22) Anmeldetag: **11.06.87**

(51) Int. Cl.5: **G01N 33/531**, G01N 33/543, //G01N33/94

(54) **Verfahren zur Herstellung eines immunreaktiven Trägermaterials für die heterogene immunologische Analyse.**

(30) Priorität: **20.06.86 DE 3620653**

(43) Veröffentlichungstag der Anmeldung: **23.12.87 Patentblatt 87/52**

(45) Bekanntmachung des Hinweises auf die Patenterteilung: **16.09.92 Patentblatt 92/38**

(84) Benannte Vertragsstaaten: **AT BE CH DE ES FR GB GR IT LI LU NL SE**

(56) Entgegenhaltungen:
EP-A- 0 098 590
EP-A- 0 185 372
GB-A- 1 420 916

ESSENTIAL IMMUNOLOGY, I.M. ROITT, 5. Aufl., 1984, Blackwell Scientific Publications, Oxford; S.4-6; Fig. 1 und 3, S. 5

(73) Patentinhaber: **BOEHRINGER MANNHEIM GMBH**
**Sandhofer Strasse 116**
**W-6800 Mannheim 31(DE)**

(72) Erfinder: **Herrmann, Uwe, Dr.rer.nat.**
**Ludwigstr. 30**
**W-8011 Kirchheim(DE)**

EP 0 249 877 B1

**Beschreibung**

Die Erfindung betrifft ein Trägermaterial für die heterogene immunologische Analyse, ein Verfahren zu seiner Herstellung und seine Verwendung.

Durch heterogene immunologische Analyse können immunologisch aktive Rezeptoren, wie z. B. Antikörper, Antigene oder Haptene, bestimmt werden. Derartige Verfahren sind dem Fachmann, beispielsweise unter den Bezeichnungen kompetitiver Test, JEMA-Test oder Sandwich-Test bekannt. Diesen Verfahren gemeinsam ist, daß einer der Reaktionspartner nicht abwaschbar an ein unlösliches Trägermaterial immobilisiert ist.

Die Fixierung eines Rezeptors an ein unlösliches Trägermaterial kann durch kovalente oder adsorptive Bindung erfolgen (Methods in Enzymology, 73b, S. 203 - 244 (1981) und B.E.T. Maggio "Enzyme Immunoassay" CAC Press, Florida 1980, insbesondere die Seiten 175 - 178).

Sowohl die kovalente als auch die adsorptive Bindung von Rezeptoren an die Festphase hat schwerwiegende Nachteile. Die bei der kovalenten Bindung erforderliche chemische Veränderung am Rezeptor bewirkt in vielen Fällen auch eine Veränderung der biologischen Aktivität des Rezeptors. Ein Nachteil der adsorptiven Bindung liegt darin, daß die Beladungsdichte sehr gering ist und beim Waschen des Trägermaterials die Desorption des Rezeptors nicht vollständig verhindert werden kann.

Eine weitere Fixierungsmethode von Rezeptoren auf ein Trägermaterial ist die Immunpräzipitat-Bildung (vgl. z. B. PCT-WO 82/02601 und US-A-3,888,629). Hierbei wird der zu immobilisierende Rezeptor mit einem präzipitierenden Partner auf einem Trägermaterial zusammengebracht und eine Immunpräzipitation durchgeführt. Zwischen dem Rezeptor und seinem immunologischen Partner bildet sich bei deren Präzipitation ein Netzwerk aus, welches in das Innere und/oder die Oberfläche des Trägermaterials eingebettet ist und durch Waschen nicht mehr abgelöst werden kann. Damit wird eine hohe Beladungsdichte ohne chemische Modifikation des zu immobilisierenden Rezeptors erreicht.

Die bekannten Verfahren zur Immunpräzipitation haben jedoch den Nachteil, daß hierbei der Rezeptor ungleichmäßig über das Trägermaterial verteilt wird. Dieser Nachteil ist besonders dann gravierend, wenn das Trägermaterial z. B. für Zwecke der quantitativen Analyse niedrig konzentrierter Haptene oder Proteine eingesetzt werden soll. Dieser Nachteil kann durch die nicht vorveröffentlichte deutsche Patentanmeldung DE-A-3446636 überwunden werden.

In der DE-A-3446636 ist ein Verfahren zur Herstellung eines immunreaktiven porösen Trägermaterials, durch Aufbringung von je einer Lösung eines ersten Partners einer Immunreaktion und eines mit diesem präzipitierenden zweiten Partners einer Immunreaktion, Inkubation des mit den Lösungen getränkten Trägermaterials zur Immunpräzipitation, ggf. waschen und anschließend trocknen des imprägnierten Trägermaterials, dadurch gekennzeichnet, daß man eine Lösung beider Partner der Immunreaktion herstellt, welche einen Hemmstoff für die Immunpräzipitation enthält, das Trägermaterial mit dieser Lösung imprägniert und dann die Immunpräzipitation durch Entfernen des Hemmstoffs oder Aufhebung der Hemmwirkung auslöst, beschrieben.

Es hat sich jedoch gezeigt, daß bei Verwendung von Trägermaterialien, die durch Immunpräzipitation immobilisierte Rezeptoren enthalten, in vielen Fällen die Präzision und die Linearität der Eichkurve (Meßsignal/Konzentration) nicht befriedigend sind.

Die Aufgabe der vorliegenden Erfindung besteht demnach darin, ein verbessertes Trägermaterial, welches einen immobilisierten Rezeptor im Immunpräzipitat enthält, herzustellen, mit dem in heterogenen immunologischen Tests eine verbesserte Präzision und eine höhere Linearität der Eichkurve erreicht werden können.

Gelöst wird diese Aufgabe erfindungsgemäß durch ein Verfahren zur Herstellung eines immunreaktiven porösen Trägermaterials für die heterogene immunologische Analyse durch Aufbringen von je einer Lösung eines Rezeptors und eines diesen immunologisch präzipitierenden Partners auf ein Trägermaterial, Inkubation des mit diesen Lösungen getränkten Trägermaterials zur Immunpräzipitation, ggf. waschen und anschließend trocknen des imprägnierten Trägermaterials, welches dadurch gekennzeichnet ist, daß man die Konzentrationen des Rezeptors und des Partners so wählt, daß bei der Imprägnierung ihr molares Verhältnis größer als das durch das Heidelberger Maximum definierte Verhältnis ist.

Überraschenderweise hat sich gezeigt, daß das Verhältnis von Rezeptor und immunpräzipitierendem Partner einen wesentlichen Einfluß auf die Präzision und die Linearität der Eichkurve hat. Besonders überraschend ist dabei, daß mit den für die Immunpräzipitation günstigen Konzentrationsverhältnisse, wie sie z. B. im Heidelberger Maximum vorliegen, nicht die optimale Präzision und Linearität der Eichkurve erreicht werden.

Die molaren Konzentrationen des Rezeptors und des immunreaktiven Partners werden so gewählt, daß ihr Verhältnis größer als das durch das Heidelberger Maximum definierte Verhältnis ist. Das Verhältnis des Heidelberger Maximums läßt sich durch Trübungstests leicht vorher festlegen. Eine entsprechende Trübungskurve findet sich beispielsweise in "Methods in Immunology and Immunochemistry", Band III, Academic Press 1971, Kapitel 13, Seite 10. Bei der Erstellung einer solchen Kurve wird die Trübung aufgetragen, die bei konstanter Menge eines der beiden Reaktionspartner mit zunehmenden Mengen des anderen Reaktionspartners erhalten wird. Das Trübungsmaximum ist das Heidelberger Maximum.

Üblicherweise liegt das Heidelberger Maximum für das molare Verhältnis Rezeptor zu präzipitierendem Partner für gereinigte Einsatzstoffe bei ca. 1 : 3,5. Die erfindungsgemäß vorteilhafte Wirkung wird dann erzielt, wenn dieses Verhältnis größer ist. Bevorzugt ist es, ein Verhältnis von größer oder gleich 1 : 3 anzuwenden. Besonders bevorzugt ist es, das Verhältnis größer als 1 : 2,5 sowie kleiner als 1 : 1 zu wählen.

Rezeptor und präzipitierender Partner können entweder sequenziell oder simultan auf das Trägermaterial aufgebracht werden. Bei der sequenziellen Methode wird das Trägermaterial zunächst entweder mit dem Rezeptor oder dem präzipitierenden Partner getränkt und anschließend der entsprechende andere Partner als Lösung zugegeben. Bevorzugt wird jedoch die Imprägnierung des Trägermaterials simultan durchgeführt. Besonders bevorzugt erfolgt die Imprägnierung in Gegenwart eines Hemmstoffes, wie in DE-A-3446636 beschrieben. Hierbei wird sowohl das "Ein-Schritt-Verfahren" als auch das "Zwei-Schritt-Verfahren" bevorzugt. Um eine Vernetzung von Rezepttor und immunpräzipitierendem Partner zu erreichen, müssen beide bzgl. ihrer immunologischen Bindefähigkeit miteinander mindestens bivalent sein. Beispielsweise kann ein Immunpräzipitat gebildet werden zwischen zwei bivalenten Antikörpern oder Antikörperfragmenten, oder einem Antikörper und einem haptenisierten Protein.

Als Rezeptoren sind demzufolge immunologisch mindestens bivalente Substanzen geeignet, wie z. B. Antikörper und deren bivalente Fragmente, die sowohl monoklonal als auch polyklonal sein können und andere Proteine. Ebenso geeignet sind Antigene oder haptenisierte Proteine. Wenn für eine immunologische Bestimmung ein immobilisiertes Hapten verwendet werden soll, so wird als Rezeptor bevorzugt ein Polyhapten eingesetzt. Dabei sind beispielsweise mehrere Haptenmoleküle, die in diesem Fall auch monovalent sein können, an ein bi- oder multivalentes Trägerprotein, wie z. B. einen Antikörper oder Serumalbumin, gebunden. Damit kann also auch für monovalente Haptene eine Immunpräzipitation erreicht werden.

Der immunpräzipitierende Partner der Reaktion muß entsprechend dem Rezeptor ausgewählt werden. Bevorzugt wird dazu ein Antikörper verwendet, der bivalent ist und gegen den Rezeptor gerichtet ist. Besonders bevorzugt wird ein Antikörper verwendet, der eine möglichst hohe Affinität zum Rezeptor hat, damit die Fixierung des Immunpräzipitats auf dem Trägermaterial möglichst fest ist.

Als Trägermaterial kommen im Rahmen der Erfindung die üblichen Festträger für immunreaktive Substanzen in Betracht. Ein derartiges Trägermaterial, welches häufig auch als Matrix bezeichnet wird, kann beispielsweise aus Glas, Kunststoff, Papier, porösem Metall oder dergleichen bestehen, vorausgesetzt, das Trägermaterial ist ausreichend von zusammenhängenden flüssigkeitsdurchlässigen Hohlräumen durchsetzt. Geeignet sind auch natürliche, künstliche, organische oder anorganische Polymere. Ebenso kommen faserartige, schwammartige oder gesinterte Substanzen in Betracht. Die Reagenzträger können flächig, partikulär oder in anderer Form eingesetzt werden. Besonders bevorzugt werden als Reagenzträger flächige, poröse Träger, wie z. B. Papier, Schaumstoff-Folien, Glasmatten oder dergleichen eingesetzt.

Die Anwendung eines erfindungsgemäß hergestellten immunreaktiven Trägermaterials im Rahmen des heterogenen Enzymimmunoassays kann z. B. so erfolgen, daß Hapten (H) oder Protein (P), das in einer Probe wie Pufferlösung, Serum, Plasma, Urin, Kulturüberstand, u. a. enthalten ist, mit einem markierten Binder (B) versetzt wird. Als Binder kommen u. a. Antikörper, Fab⁻-Fragment bzw. Fab-Fragment sowie Liganden in Frage, welche spezifisch mit dem Hapten oder Protein reagieren. Als Markierung des Binders kann z. B. ein Enzym, Fluoreszenz-Label oder Radioisotop, verwendet werden, in den nachfolgenden Beispielen wurde die ß-Galactosidase gewählt. Die Menge des zugegebenen Binders kann molmäßig sowohl im Überschuß als auch im Unterschuß zu dem in der Probe vorhandenen Hapten oder Protein vorliegen.

Diese Mischung wird für eine konstante Zeit inkubiert, während der sich die Komplexe H-B bzw. P-B bilden. Nach Ablauf dieser Zeitspanne liegen im Reaktionsgemisch drei Spezies vor, nämlich der Komplex, bestehend aus Hapten/Protein, und Binder (H-B, P-B), restliches freies Hapten/Protein (H/P) und restlicher freier Binder (B).

Die Trennung dieser Spezies erfolgt in einem zweiten Schritt mittels Immunosorption. Dazu wird das Gemisch auf die erfindungsgemäß hergestellte Immunpräzipitation-Festphase aufgetragen, die das nachzuweisende Hapten bzw. den Antikörper gegen das zu bestimmende Protein gebunden enthält.

- Beim Hapten-Test bindet nunmehr der freie Binder, nicht aber der mit Hapten abgesättigte Binder an die Festphase. Folglich enthält der Überstand bzw. das Eluat der Festphase den mit dem Hapten der Probe abgesättigten Binder. Die quantitative Bestimmung des Haptens erfolgt nunmehr über die Markierung des Binders, in den vorliegenden Beispielen über die Bestimmung der ß-Galactosidase mittels o-Nitrophenyl-ß-D-galactosid oder Chlorphenolrot-ß-D-galactosid.

- Beim Protein-Test bindet der Komplex aus Protein und Binder, nicht jedoch der freie Binder an die Festphase, Reste des freien Binders werden durch Waschen vom erfindungsgemäß hergestellten Immunosorbens entfernt. Der quantitative Nachweis des Proteins erfolgt über den markierten Binder über die Bestimmung der ß-Galactosidase mittels o-Nitrophenyl-ß-D-galactosid oder Chlorphenolrot-ß-D-galactosid.

Eine andere Anwendung sind kompetitive Immuntests. Der Einsatz des erfindungsgemäß hergestellten immunreaktiven Trägermaterials kann so erfolgen, daß der Analyt (Hapten oder Protein), der in der Probe enthalten ist, mit einer konstanten Menge an markiertem Analyten versetzt wird. Die Markierung kann z. B. ein Enzym, ein Fluoreszenz-Label, Radioisotop und anderes sein. Diese Mischung wird auf die Matrix gegeben. Auf der Matrix ist ein Antikörper immobilisiert, welcher gegen den Analyten gerichtet ist. Die Mischung wird auf der Matrix eine bestimmte Zeit inkubiert. Während dieser Zeit konkurrieren sowohl unveränderter Analyt als auch markierter Analyt um die Bindungsstellen auf der Matrix. Je mehr Analyt in der Probe vorhanden ist, desto weniger markierter Analyt wird von der Matrix gebunden und umgekehrt. Am Ende der Inkubationsphase wird die Flüssigkeit aus der porösen Matrix entfernt, z. B. durch Zentrifugieren. Bestimmt wird dann die Menge des markierten Analyten entweder in der freien Phase oder die Menge des an die Matrix gebundenen markierten Analyten.

Eine weitere Anwendung der erfindungsgemäß hergestellten Trägermaterialien kann so erfolgen, daß der Analyt (Hapten oder Protein) mit einer konstanten Menge an markiertem Antikörper, der gegen den Analyten gerichtet ist, versetzt wird. Mögliche Arten der Markierung des Antikörpers wurden weiter oben beschrieben. Diese Mischung wird entweder eine bestimmte Zeit inkubiert und dann auf das poröse Trägermaterial gegeben oder alternativ sofort nach Mischung auf das poröse Trägermaterial gegeben. Wenn der Analyt ein Hapten ist, enthält das Trägermaterial das nachzuweisende Hapten oder ein Derivat davon in fixierter Form, falls der Analyt ein Protein ist, enthält das Trägermaterial das nachzuweisende Protein oder ein Derivat davon ebenfalls in fixierter Form. Wurde die erste Mischung vor Auftragung auf das Trägermaterial inkubiert, so bindet markierter Antikörper mit noch freien Bindungsstellen an das poröse Trägermaterial. Wurde die Mischung sofort auf das Trägermaterial gegeben, so konkurrieren der Analyt aus der Probe und der an das Trägermaterial fixierte Analyt um die Bindungsstellen des markierten Antikörpers. Am Ende der Inkubationsphase wird die Flüssigkeit aus dem porösen Trägermaterial entfernt und die Menge des markierten Antikörpers entweder in der flüssigen Phase oder auf dem porösen Trägermaterial bestimmt.

Die folgenden Beispiele erläutern die Erfindung weiter. Gewinnung der für den Test verwendeten Einsatzstoffe und Testführung (vgl. auch DE-A-3446636):

A) Herstellung von Polyhaptenen (PH):

Die Herstellung von PH ist Stand der Technik. So kann z. B. ein Digoxin- oder Diphenyl-Hydantoin-PH über reaktive unsymmetrische Dicarbonsäureester/aktivierte Haptenester und deren Bindung an ein Trägerprotein gewonnen werden.

Die Auswahl der Trägerproteine unterliegt keinen Einschränkungen, sofern nur ein entsprechender "fällender" Antikörper zur Vergügung steht bzw. hergestellt werden kann.

Die Herstellung von Theophyllin-PH kann beispielsweise über Theophyllin-7-Buttersäure (Herstellung nach EPS 00 13 910) oder über Theophyllin-7-Propionsäure und dessen Kopplung an RSA oder andere Proteine erfolgen. (Nishikawa, Clin. Chim. Acta 91, 1979, 59-65, Erlenger, J. Biol. chem. 228, 1975, 713-727)

B) Herstellung des Binders (Beispiel Theophyllin)

Die Herstellung des Immunogens sowie des Antiserums kann z. B. erfolgen nach JA-OS 57-099598, DE-OS 28 05 961, EP 00 13 910, EP 00 98 179, EP 0077 896, Res. Commun. Chem. Pathol. Pharmacol. 13, 1976, 497-505 oder Clin. Chim. Acta 91, 1979, 59 - 65.

Soweit eine immunsorptive Aufreinigung von Antigen und Antikörper erfolgte, wurde als Träger das "Affinitätsadsorbens, Glutardialdehyd aktiviert" von Boehringer Mannheim (Best. Nr. 665525) verwendet. Für die unten beschriebenen Beispiele wurde nach Vorschrift des Herstellers für die Antigen-Aufreinigung ein Schaf-Antikörper gegen Kaninchen-IgG bzw. ein spez. Antikörper gegen das Hapten und für die Antikörper-Aufreinigung Kaninchen IgG an den Träger gebunden. Die Durchführung der Immunsorption erfolgte, wie in der Arbeitsanleitung zum Affinitätsadsorbens beschrieben.

Antiserum, gerichtet gegen Theophyllin, wurde über Ammoniumsulfat-Fällung und Passage über DEAE-Cellulose zur IgG-Fraktion aufgereinigt. Die Papain-Spaltung wurde nach R.R. Porter, Biochem. J. 73, 119-126 (1959) durchgeführt.

4

Die Fab-Fragmente wurden von den nicht verdauten IgG-Molekülen und den Fc-Fragmenten mittels Gelfiltration über Sephadex® G 100 und Ionenaustauschchromatographie über DEAE-Cellulose nach Literaturvorschrift (K. Malinowski und W. Manski in "Methods in Enzymology"; J.J. Langone und H. van Vunakis eds., Academic Press, Vol. 73 (1981) pp. 418-459) abgetrennt. Die resultierende Fab-Fraktion wurde chromatographisch gereinigt und an ß-Galactosidase nach der Vorschrift von T. Kitiwaga in "Enzyme Immunoassay; Ishikawa, T. Kawai, K. Miyai, eds. Igaku Shoin, Tokyo/New York (1981) pp. 81-89, gekoppelt.

C) Herstellung der Schafantikörper gegen Kaninchen- bzw bzw. Maus-IgG/Fc$\gamma$

Kaninchen-Schlachtserum bzw. Mäuseserum wurde einer Ammonsulfat-Fällung unterzogen. Nach Passage über DEAE-Cellulose und Papain-Spaltung, Gel- und Ionenaustauschchromatographie (siehe B)) erhält man die Fc-Fragmente des Kaninchen- bzw. Maus-IgG's als Immunogene.

Die Aufarbeitung der Schaf-Antiseren erfolgt wie unter B) beschrieben.

D) Testführung (Beispiel Theophyllin):

Theophyllin-Standards in einem Rinderserumalbumin-haltigen Puffer wurden 1 : 100 mit 0,9 % NaCl-Lösung verdünnt. Zu 200 $\mu$l verdünntem Standard wurde 200 $\mu$l Binder (1,6 U/ml, bestimmt mit ortho-nitrophenyl-ß-D-galactosid), hergestellt wie unter B) beschrieben, in PBS pH 7,8 (Phosphate buffered saline) bei 37°C inkubiert.

Danach werden 50 $\mu$l des Gemisches auf 1 cm$^2$ des flächigen immunreaktiven Trägermaterials gegeben. Man inkubiert weitere 5 Minuten bei 37°C und zentrifugiert 1 Minute mit einer Eippendorf-Zentrifuge.

Die Enzymaktivität der Theophyllin-Binder-Komplexe in der freien Phase kann nunmehr kinetisch durch Zusatz von Ortho-nitro-phenyl-ß-D-Galactosid bei 410 nm gemessen werden.

**Beispiel 1**

Herstellung von Theophyllin-Immunpräzipitat-Vliesen.

Als Antigen wurde verwendet: Polyhapten (PH), bestehend aus Kaninchen-IgG und daran gebundenem Theophyllin (Molverhältnis IgG:Theophyllin = 1:3). Das Antigen wurde mittels Ionenaustauschchromatographie aufgereinigt.

Als fällender Antikörper (FAK) wurde verwendet: polyklonaler Antikörper, gerichtet gegen den Fc-Teil des Kaninchen-IgG's. Der Antikörper wurde immunsorptiv über eine Kaninchen-IgG-Säule aufgereinigt.

Als poröses Trägermaterial (Vlies) wurde verwendet: Mischvlies aus Zellwolle und Polyester (Firma Kalff, Euskirchen, Deutschland), oder aus Linters, Zellwolle, Polyamid, (Firma Binzer, Hatzfeld, Eder, Deutschland).

Antigen (c = 1,5 mg/ml) und Antikörper (c = 3 mg/ml Verhältnis 1 : 2 bzw. 5,25 mg/ml Verhältnis 1 : 3,5 Heidelberger Maximum) werden getrennt in 50 mM/l Essigsäure aufgenommen. Dann läßt man eine Stunde bei Raumtemperatur stehen. Die Lösungen werden im Verhältnis 1:1 gemischt. In einem nachfolgenden Schritt wird die Lösung mit 4 Teilen 50 mM/l Essigsäure + 100 mM/l Natriumchlorid verdünnt. Die Lösung wird nochmals 30 Minuten bei Raumtemperatur stehengelassen. Anschließend wird das Vlies durch die Tränklösung gezogen und 30 Minuten bei 30°C im Umlufttrockenschrank getrocknet.

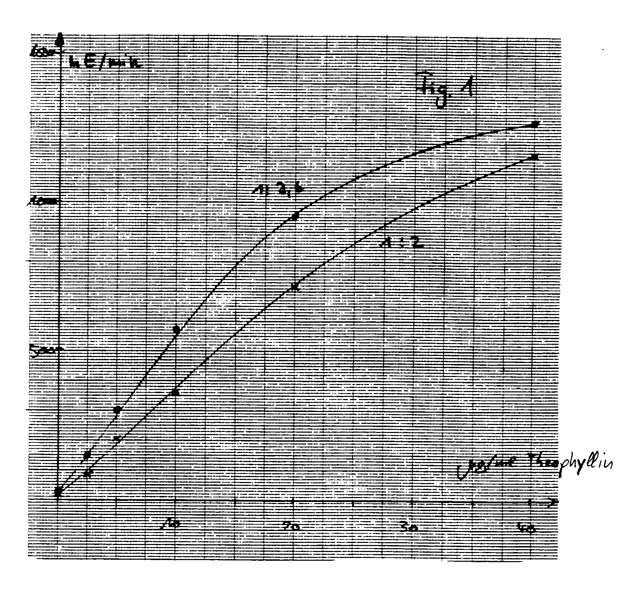

Die erreichte Linearisierung der Eichkurve ist in Figur 1 dargestellt. Verglichen mit den Konzentrationsverhältnissen am Heidelberger Maximum, wurde mit einem Verhältnis der Reaktionspartner von 1:2 die Präzision der Messung eines Theophyllin-Standards von 10 $\mu$g/ml von 6 % Variationskoeffizient auf ca. 3 % Variationskoeffizient verbessert.

**Beispiel 2**

Aufnahme einer Heidelbergerkurve für Theophyllin

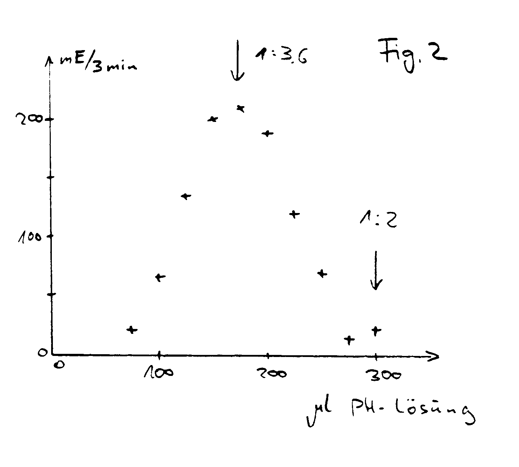

In einer Küvette wird jeweils ein konstantes Volumen einer bekannten Konzentration an fällendem AK vorgelegt (4,1 mg/ml) und mit steigenden Volumina einer Antigenlösung (PH-Lösung) bekannter Konzentration (2,6 mg/ml) versetzt. Es wird die Kinetik der Trübungszunahme im Photometer bei $\lambda$ = 340 nm beobachtet. Das Heidelberger Maximum entspricht dem Konzentrationsverhältnis mit der schnellsten Trübungsänderung. Aus Fig. 2 ergeben sich das Heidelberger Maximum bei einem Verhältnis von:

$$\frac{c\ (FAK)\ x\ v\ (FAK)}{c\ (PH)\ x\ v\ (PH)} = \frac{4,1\ .\ 400}{2,6\ .\ 175} = \frac{1}{3,6} \quad mg\ FAK/mg\ PH$$

**Beispiel 3**

Herstellung von Diphenylhydantoin (DPH)-Immunpräzipitat-Vliesen.

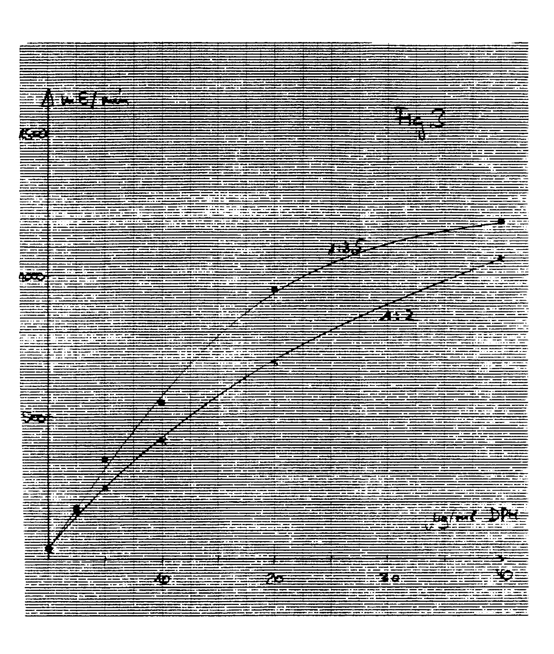

Die Herstellung von Diphenylhydantoin-Immunpräzipitat-Vliesen erfolgt analog Beispiel 1. Die erreichte Linearisierung der Eichkurve ist in Fig. 3 dargestellt.

**Patentansprüche**

1. Verfahren zur Herstellung eines immunreaktiven porösen Trägermaterials für die heterogene immunologische Analyse durch Aufbringen von je einer Lösung eines Rezeptors und eines diesen immunologisch präzipitierenden Partners auf ein Trägermaterial, Inkubation des mit den Lösungen getränkten Trägermaterials zur Immunpräzipitation, ggf. waschen und anschließend trocknen des imprägnierten Trägermaterials, dadurch gekennzeichnet, daß man die Konzentrationen des Rezeptors und des Partners so wählt, daß bei der Imprägnierung ihr molares Verhältnis größer als das durch das Heidelberger Maximum definierte Verhältnis ist.

2. Verfahren nach Anspruch 1, dadurch gekennzeichnet, daß Rezeptor und Partner im molaren Verhältnis größer oder gleich 1 : 3 verwendet werden.

3. Verfahren nach Anspruch 1 - 2, dadurch gekennzeichnet, daß Rezeptor und Partner im molaren Verhältnis 1 : 2,5 bis 1 : 1 verwendet werden.

4. Verfahren nach Anspruch 1 - 3, dadurch gekennzeichnet, daß man je eine Lösung des Rezeptors und des Partners verwendet, wobei mindestens eine der Lösungen einen Hemmstoff für die Immunpräzipitation enthält, das Trägermaterial mit diesen Lösungen imprägniert und dann die Immunpräzipitation durch Entfernen des Hemmstoffs oder Aufhebung der Hemmwirkung auslöst.

5. Verfahren nach Anspruch 1 - 4, dadurch gekennzeichnet, daß als Rezeptor ein Protein und als Partner ein gegen dieses Protein gerichteter Antikörper oder ein Fragment davon verwendet wird.

6. Verfahren nach Anspruch 5, dadurch gekennzeichnet, daß man als Rezeptor ein Protein verwendet, an das mindestens ein Hapten oder Antigen gekoppelt ist.

7. Verfahren nach Anspruch 6, dadurch gekennzeichnet, daß man als Protein einen Antikörper oder ein Fragment davon verwendet, an den mindestens ein Hapten oder Antigenmolekül gekoppelt ist.

8. Trägermaterial für die heterogene immunologische Analyse, dadurch gekennzeichnet, daß es einen Rezeptor und einen immunologisch präzipitierenden Partner in einem molaren Verhältnis, welches größer als das durch das Heidelberger Maximum definierte Verhältnis ist, enthält.

9. Trägermaterial nach Anspruch 8, dadurch gekennzeichnet, daß das Verhältnis Rezeptor zu Partner größer oder gleich 1 : 3 ist.

10. Trägermaterial nach Anspruch 9, dadurch gekennzeichnet, daß das molare Verhältnis Rezeptor zu Partner zwischen 1 : 2,5 und 1 : 1 beträgt.

11. Verwendung eines Trägermaterials gemäß den Ansprüchen 8 bis 10 zur heterogenen immunoligschen Analyse.

**Claims**

# EP 0 249 877 B1

1. Process for the production of an immune-reactive, porous support material for heterogeneous immunological analysis by application to a support material of a solution of a receptor and of a partner precipitating this immunologically, incubation of the support material impregnated with these solutions for the immune precipitation, possible washing and subsequent drawing of the impregnated carrier material, characterised in that one so selects the concentrations of the receptor and of the partner that, in the case of the impregnation, their molar ratio is greater than the ratio defined by the Heidelberger maximum.

2. Process according to claim 1, characterised in that the receptor and partner are used in a molar ratio greater than or equal to 1:3.

3. Process according to claim 1 - 2, characterised in that the receptor and partner are used in the molar ratio of 1:2.5 to 1:1.

4. Process according to claim 1 - 3, characterised in that one uses a solution of each of the receptor and of the partner, whereby at least one of the solutions contains an inhibitor for the immune precipitation, impregnates the support material with these solutions and then initiates the immune precipitation by removal of the inhibitor or removal of the inhibiting action.

5. Process according to claim 1 - 4, characterised in that, as receptor, one uses a protein and, as partner, an antibody directed against this protein or a fragment thereof.

6. Process according to claim 5, characterised in that, as receptor, one uses a protein to which is coupled at least one hapten or antigen.

7. Process according to claim 6, characterised in that, as protein, one uses an antibody or a fragment thereof to which is coupled at least one hapten or antigen molecule.

8. Support material for heterogeneous, immunological analysis, characterised in that it contains a receptor and an immunologically precipitating partner in a molar ratio which is greater than the ratio defined by the Heidelberger maximum.

9. Support material according to claim 8, characterised in that the ratio of receptor to partner is greater than or equal to 1:3.

10. Support material according to claim 9, characterised in that the molar ratio of receptor to partner amounts to between 1:2.5 and 1:1.

11. Use of a support material according to claims 8 to 10 for heterogeneous, immunological analysis.

**Revendications**

1. Procédé pour la préparation d'un matériau de support poreux immunoréactif pour l'analyse immunologique hétérogène, consistant à déposer, sur un matériau de support, un récepteur et son partenaire de précipitation immunologique, chacun à partir d'une solution, à incuber le matériau de support imprégné des solutions pour réaliser l'immunoprécipitation, éventuellement à laver et ensuite, à sécher le matériau de support imprégné, caractérisé en ce que la concentration du récepteur et la concentration de son partenaire sont choisies de façon que lors de l'imprégnation, leur rapport molaire soit supérieur au rapport défini par le maxima de Heidelberger.

2. Procédé selon la revendication 1, caractérisé en ce que le récepteur et son partenaire sont utilisés dans un rapport molaire supérieur ou égal à 1 : 3.

3. Procédé selon les revendications 1 - 2, caractérisé en ce que le récepteur et son partenaire sont utilisés dans un rapport molaire de 1 : 2,5 à 1 : 1.

4. Procédé selon les revendications 1 - 3, caractérisé en ce que l'on utilise une solution d'un récepteur et une solution de son partenaire, l'une au moins de ces solutions contenant un inhibiteur de l'immunopré-

7

cipitation, on imprègne le matériau de support avec cette solution et ensuite, on déclenche l'immunoprécipitation par élimination de l'inhibiteur ou par suppression de l'action inhibitrice.

5. Procédé selon les revendications 1 - 4, caractérisé en ce que comme récepteur, on utilise une protéine et comme partenaire, un anticorps ou un fragment d'anticorps dirigé contre cette protéine.

6. Procédé selon la revendication 5, caractérisé en ce que comme récepteur, on utilise une protéine à laquelle est couplé au moins un haptène ou antigène.

7. Procédé selon la revendication 6, caractérisé en ce que comme protéine, on utilise un anticorps ou un fragment d'anticorps auquel est couplé au moins un haptène ou une molécule d'antigène.

8. Matériau de support pour l'analyse immunologique hétérogène, caractérisé en ce qu'il contient un récepteur et un partenaire de précipitation immunologique dans un rapport molaire qui est supérieur au rapport défini par le maxima de Heidelberger.

9. Matériau de support selon la revendication 8, caractérisé en ce que, le rapport du récepteur au partenaire est supérieur ou égal à 1 : 3.

10. Matériau de support selon la revendication 9, caractérisé en ce que le rapport molaire du récepteur au partenaire est compris entre 1 : 2,5 et 1 : 1.

11. Utilisation d'un matériau de support selon les revendications 8 à 10, pour l'analyse immunologique hétérogène.

Fig. 1

Fig. 2

Fig. 3